# EUROPEAN PATENT APPLICATION

(11) **EP 4 582 054 A1**
(43) Date of publication of application: **09.07.2025**
(21) Application number: 23859339.6
(22) Date of filing: 29.08.2023
(51) Int. Cl.: A61F 2/24

(54) **PROSTHESIS SYSTEM**

(30) Priority: 29.08.2022 CN 202211043537; 29.08.2022 CN 202222309623 U
(71) Applicant: Enlight Medical Technologies (Shanghai) Co., Ltd., Shanghai 201318 (CN)
(72) Inventor: ZHOU, Guanguan, Shanghai 201315 (CN); LI, Wensi, Shanghai 201315 (CN); HU, Zhanming, Shanghai 201315 (CN); LI, Fang, Shanghai 201315 (CN)
(74) Representative: Balder IP Law, S.L.
(86) International application number: PCT/CN2023/115484
(87) International publication number: WO 2024/046309

(57) **Abstract**

Disclosed is a prosthesis system, comprising a delivery device (100) and a prosthesis. The delivery device (100) comprises a first connecting shaft (110) and a first control wire (120). The prosthesis comprises a first base (210) and a first movable member, wherein the first base (210) is detachably coupled to the first connecting shaft (110), forming a first preset channel (300) at the coupling point. The first control wire (120) is provided with a first segment (121) for independently driving the movement of the first movable member and a second segment (122), which are connected in sequence, wherein when the first connecting shaft (110) and the first base (210) are coupled, the first preset channel (300) is in a closed state, and the second segment (122) is constrained within the first preset channel (300), thereby constraining the first segment (121) at the coupling point; when the first connecting shaft (110) and the first base (210) are detached, the first preset channel (300) is in a non-closed state, and the first control wire (120) can be separated from the first preset channel (300). The prosthesis system of the present application can reduce the manufacturing cost of the prosthesis system under the condition that the delivery device (100) can safely and reliably adjust the movable member in the prosthesis.

## Description

### CROSS-REFERENCE TO RELATED APPLICATIONS

The present application is based on Chinese patent application No. 2022110435377 filed on August 29, 2022 and Chinese patent application No. 202222309623X filed on August 29, 2022, and claims priority to these two Chinese patent applications. The entire contents of these two Chinese patent applications are hereby incorporated by reference into the present application.

### TECHNICAL FIELD

The present disclosure relates to the technical field of medical devices, and in particular to a prosthesis system.

### BACKGROUND

Traditional surgeries (such as open surgery) has disadvantages of large incision (i.e. long incision, generally greater than or equal to 10 centimeters, resulting in a linear scar after incision healing, which effects the aesthetics), strong and long-lasting pain (i.e. incision is often accompanied by postoperative pain), slow recovery (large surgical incision easily causes damage to muscles, blood vessels, and corresponding nerves near the incision, which may be potentially accompanied by some tissue infection complications, resulting in slow recovery). As a result, minimally invasive surgery emerged as an alternative. Minimally invasive surgery has advantages of small incision (i.e. the incision length is generally in the range of 0.5 centimeters to 1 centimeter), mild pain (due to small incision, which causes less pain to the patient), and fast recovery (due to significant reduction in organ damage and interference with organ function, it is less likely to cause complications and shortens postoperative recovery time).

**In** the existing minimally invasive surgeries, a delivery device is generally utilized to deliver a prosthesis to a target position in a human body (i.e. the location where the surgery needs to be performed) through a surgical path such as an anatomy lumen, and then a control wire of the delivery device is manipulated to adjust a movable member in the prosthesis to change its state, finally achieving the implantation of the prosthesis. US patent document US20210145574A1 discloses an embodiment of adjusting the movable member of a prosthesis by using a control wire to change the state of the movable member. According to the US patent document US20210145574A1, one end of the control wire has a protrusion, and the other end thereof passes through a hole in the movable member and extends to a proximal end of the delivery device for the operator to adjust the position and/or posture of the movable member through the control wire; a connecting shaft of the delivery device is provided with a through-hole, and the diameter of one end of the through-hole is smaller than the size of the protrusion, and the diameter of the other end of the through-hole is larger than the size of the protrusion. Therefore, the position of the protrusion is limited by the smaller-diameter end of the through hole. When the control wire needs to be withdrawn, the protrusion can be withdrawn from the other larger- diameter end of the through hole.

However, the structure of such the connecting shaft is relatively complex, and the processing of the connecting shaft is difficult, resulting in higher manufacturing costs for the delivery device, which in turn results in high manufacturing costs for a prosthesis system with this type of delivery device. Therefore, there is an urgent need to provide a prosthesis system that has a simple structure and reduced manufacturing cost, while the delivery device of the prosthesis system can safely and reliably adjust the movable member of the prosthesis.

### SUMMARY

It is an objective of the present disclosure to provide a prosthesis system with a reduced manufacturing cost while the delivery device can safely and reliably adjust the movable member in the prosthesis.

To solve the above problems, an embodiment of the present disclosure provides a prosthesis system including a delivery device and a prosthesis. The delivery device includes a first connecting shaft and a first control wire. The prosthesis includes a first base and a first movable member, where the first base is detachably coupled to the first connecting shaft, forming a first preset channel at a coupling site. The first control wire is provided with a first segment for independently driving movement of the first movable member and a second segment, which are connected in sequence. When the first connecting shaft and the first base are coupled, the first preset channel is in a closed state, and the second segment is constrained within the first preset channel, thereby constraining the first segment at the coupling site. When the first connecting shaft and the first base are detached, the first preset channel is in a non-closed state, and the first control wire can be detached from the first preset channel.

Another embodiment of the present disclosure provides a prosthesis system including a delivery device and a prosthesis. The delivery device includes a second connecting shaft, a second central rod, and a first control wire. The prosthesis includes a second base and a first movable member, where when the second central rod is positioned at a coupling site between the second connecting shaft and the second base, the second base is coupled to the second connecting shaft. The first control wire is provided with a first segment for independently driving movement of the first movable member and a second segment, which are connected in sequence. When the second central rod is positioned at the coupling site between the second connecting shaft and the second base, the second central rod forms at the coupling site a second preset channel in a closed state with the second connecting shaft and/or the second base, and the second segment is constrained within the second preset channel, thereby constraining the first segment at the coupling site. When the second central rod is positioned outside the coupling site between the second connecting shaft and the second base, the second preset channel is in a non-closed state, and the first control wire can be detached from the second preset channel.

The prosthesis system provided in some embodiments of the present disclosure, during the implementation of prosthesis implantation surgery, causes the preset channel to be in a closed state to constrain the second segment of the first control wire inside the preset channel, so that a distal end of the first control wire is constrained at the coupling site. Then, after the connecting shaft delivers the prosthesis to a target position in the human body through a surgical path such as an anatomy lumen, the first segment of the first control wire is operated to adjust the first movable member of the prosthesis to change the state of the prosthesis, finally achieving the implantation of the prosthesis. When it is required to detach the delivery device from the prosthesis, the base of the prosthesis is detached from the connecting shaft of the delivery device, so that the preset channel is in a non-closed state. In the non-closed state, the second segment can be detached from the preset channel, so that the first control wire can be detached from the preset channel, facilitating separation of the first control wire from the prosthesis. Then, the first control wire is withdrawn from the human body, and the connecting shaft is then withdrawn from the human body to complete the implantation surgery. In this way, the delivery device of the prosthesis system can safely and reliably adjust the movable member in the prosthesis, change the state of the prosthesis, achieve implantation of the prosthesis, and make the operation of the prosthesis system safer and more reliable.

Since the preset channel in the prosthesis system provided by the embodiments of the present disclosure has closed and non-closed states and works in conjunction with the first control wire to complete relevant surgical operations, the manufacture of the preset channel is not as difficult as the manufacture of the through-hole in the prior art with different sizes at two ends, which can reduce the manufacturing cost of the prosthesis system.

### BRIEF DESCRIPTION OF THE DRAWINGS

Fig. 1 is a schematic diagram of the coupling of a delivery device at the distal end with a mitral valve clip provided in an embodiment of the present disclosure;
Fig. 2 is a schematic diagram of the movement of a gripper of the mitral valve clip coupled with the delivery device provided in an embodiment of the present disclosure;
Fig. 3 is a longitudinal schematic diagram of a first preset channel formed by a first connecting shaft and a first base provided in an embodiment of the present disclosure;
Fig. 4 is a schematic structural exploded view of the first connecting shaft and the first base provided in an embodiment of the present disclosure;
Fig. 5 is a schematic structural exploded view of another the first connecting shaft and the first base provided in an embodiment of the present disclosure;
Fig. 6 is a schematic structural exploded view of yet another the first connecting shaft and the first base provided in an embodiment of the present disclosure;
Fig. 7 is a longitudinal cross-sectional view of the first preset channel formed by the first connecting shaft and the first base provided in an embodiment of the present disclosure;
Fig. 8 is a longitudinal cross-sectional view of another first preset channel formed by the first connecting shaft and the first base provided in an embodiment of the present disclosure;
Fig. 9 is a schematic structural diagram of a control wire provided in an embodiment of the present disclosure;
Fig. 10 is a partially enlarged view of portion a in Fig. 9;
Fig. 11 is a schematic structural diagram of another control wire provided in an embodiment of the present disclosure;
Fig. 12 is a partially enlarged view of portion b in Fig. 11;
Fig. 13 is a structural diagram of a gripper and the control wire when they are coupled, provided in an embodiment of the present disclosure;
Fig. 14 is a schematic structural diagram of another gripper and control wire when they are coupled, provided in an embodiment of the present disclosure;
Fig. 15 is a schematic structural diagram of yet another gripper and control wire when they are coupled, provided in an embodiment of the present disclosure;
Fig. 16 is a longitudinal schematic diagram of a second preset channel formed by a second central rod and a second connecting shaft or a second base provided in another embodiment of the present disclosure;
Fig. 17 is a schematic structural exploded view of the second central rod, the second connecting shaft and the second base provided in another embodiment of the present disclosure;
Fig. 18 is a longitudinal schematic diagram of the second preset channel formed by the second central rod, the second connecting shaft or the second base provided in another embodiment of the present disclosure;
Fig. 19 is a schematic structural exploded view of another second central rod, second connecting shaft, and second base provided in another embodiment of the present disclosure; and
Fig. 20 is a schematic structural exploded view of yet another second central rod, second connecting shaft and second base provided in another embodiment of the present disclosure.

### DETAILED DESCRIPTION OF THE EMBODIMENTS

Those of ordinary skill in the art may understand that in the embodiments of the present disclosure, many technical details are set forth in order to make the reader better understand the present disclosure. However, the technical solutions claimed in the present disclosure can still be achieved based on various changes and modifications of the following embodiments.

In the embodiments of the present disclosure, "proximal end" or "proximal side" refers to the end closer to the operator and farther away from the patient. Correspondingly, "distal end" or "distal side" refers to the end closer to the patient and farther away from the operator. In the embodiments of the present disclosure, the terms "parallel" and "vertical" should not be narrowly interpreted as 0° or 90°, but should be understood as reasonable angle ranges including 0° or 90° with some permissible flotation, provided that the technical effects are achieved.

An embodiment of the present disclosure provides a prosthesis system including a delivery device and a prosthesis. The delivery device includes a connecting shaft and a first control wire. The prosthesis includes a base and a first movable member, where the base is detachably coupled to the connecting shaft, forming a preset channel at a coupling site. The first control wire is provided with a first segment for independently driving movement of the first movable member and a second segment, which are connected in sequence. When the connecting shaft and the base are coupled, the preset channel is in a closed state, and the second segment is constrained within the preset channel, thereby constraining the first segment at the coupling site. When the connecting shaft and the base are detached, the preset channel is in a non-closed state, and the first control wire can be detached from the preset channel.

The prosthesis system provided in some embodiments of the present disclosure, during the implementation of prosthesis implantation surgery, causes the preset channel to be in a closed state to constrain the second segment of the first control wire inside the preset channel, so that a distal end of the first control wire is constrained at the coupling site. Then, after the connecting shaft delivers the prosthesis to a target position in the human body through a surgical path such as an anatomy lumen, the first segment of the first control wire is operated to adjust the first movable member of the prosthesis to change the state of the prosthesis, finally achieving the implantation of the prosthesis. When it is required to detach the delivery device from the prosthesis, the base of the prosthesis is detached from the connecting shaft of the delivery device, so that the preset channel is in a non-closed state. In the non-closed state, the second segment can be detached from the preset channel, so that the first control wire can be detached from the preset channel, facilitating separation of the first control wire from the prosthesis. Then, the first control wire is withdrawn from the human body, and the connecting shaft is then withdrawn from the human body to complete the implantation surgery. In this way, the delivery device of the prosthesis system can safely and reliably adjust the movable member in the prosthesis, change the state of the prosthesis, achieve implantation of the prosthesis, and make the operation of the prosthesis system safer and more reliable.

On the other hand, since the preset channel in the prosthesis system provided in some embodiments of the present disclosure has closed and non-closed states and works in conjunction with the first control wire to complete relevant surgical operations, the manufacture of the preset channel is not as difficult as the manufacture of the through-hole in the prior art with different sizes at two ends, which can reduce the manufacturing cost of the prosthesis system.

In order to make the objects, technical schemes and advantages of the embodiments of the present disclosure clearer, the embodiments of the present disclosure are described in detail below with reference to the accompanying drawings.

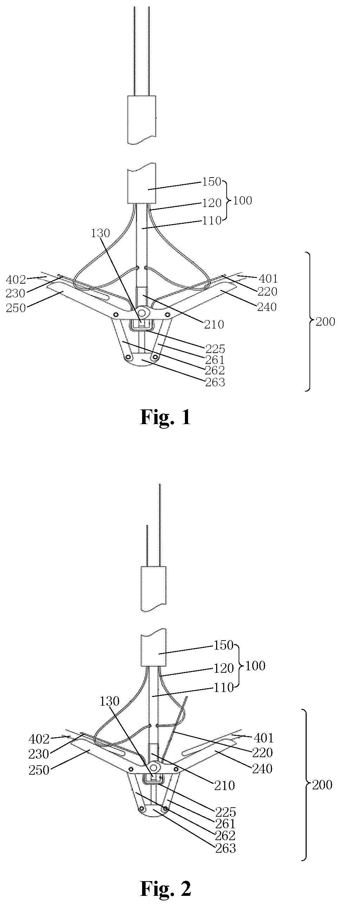

Referring to Figs. 1 to 15, some embodiments of the present disclosure provide a prosthesis system including a delivery device 100 and a prosthesis. The delivery device 100 includes a first connecting shaft 110 and a first control wire 120. The prosthesis includes a first base 210 and a first movable member, where the first base 210 is detachably coupled to the first connecting shaft 110, forming a first preset channel 300 at a coupling site. The first control wire 120 is provided with a first segment 121 for independently driving movement of the first movable member and a second segment 122, which are connected in sequence. When the first connecting shaft 110 and the first base 120 are coupled, the first preset channel 300 is in a closed state, and the second segment 122 is constrained within the first preset channel 300, thereby constraining the first segment 121 at the coupling site. When the first connecting shaft 110 and the first base 120 are detached, the first preset channel 300 is in a non-closed state, and the first control wire 120 can be detached from the first preset channel 300.

**In** this way, when a prosthesis needs to be implanted, the first connecting shaft 110 is coupled to the first base 210, forming the first preset channel 300 that is a closed state and constrains the second segment 122. The first segment 121 is constrained at the coupling site, and then the prosthesis is delivered into the human body. When the prosthesis is close to the target position in the human body, the first movable member of the prosthesis is adjusted by operating the proximal end of the first segment 121, so that the state of the prosthesis changes, further achieving the implantation of the prosthesis. When it is required to detach the delivery device 100 from the prosthesis, the first connecting shaft 110 and the first base 210 are detached to make the first preset channel 300 in a non-closed state, so that the first control wire 120 can directly free from the constraint of the first preset channel 300, and then the first control wire 120 can be withdrawn from the human body.

When the first control wire 120 is required to be withdrawn from the human body after the separation of the delivery device 100 from the prosthesis, it is achieved by placing the first preset channel 300 in a non-closed state which allows the second segment 122 to be detached from the first preset channel 300, thereby freeing the first control wire 120 from the constraint of the first preset channel 300. Thus, the first control wire 120 in no way gets stuck on the first connecting shaft 110, and the likelihood of unexpected incidents during surgery also reduce.

It should be noted that the closed state of the first preset channel 300 refers to that the first preset channel 300 has a closed shape in its circumferential direction; and the non-closed state of the first preset channel 300 refers to that the shape of the first preset channel 300 is divided into multiple non-closed parts in its circumferential direction.

**In** some embodiments, when the first segment 121 is constrained at the coupling site, the first segment 121 is adjacent to or partially located in the first preset channel 300 disposed at the coupling site. That is, when the first segment 121 is constrained at the coupling site, the first segment 121 is adjacent to the first preset channel 300, or when the first segment 121 is constrained at the coupling site, at least a portion of the connection between the first segment 121 and the second segment 122 is located within the first preset channel 300.

**In** some embodiments, the first preset channel 300 is disposed at an angle to the axis of the first connecting shaft 110 at the coupling site between the first connecting shaft 110 and the first base 210. That is, an extension direction of the first preset channel 300 has an angle greater than 0° and less than 180° with the axis of the first connecting shaft 110, and the first preset channel 300 is disposed at the coupling site between the first connecting shaft 110 and the first base 210. In an embodiment, the extension direction of the first preset channel 300 is perpendicular to the axis of the first connecting shaft 110.

In some embodiments, a wall surface of the first preset channel 300 includes a first component and a second component, where the first component is formed on the first connecting shaft 110, the second component is formed on the first base 210, and the first component and the second component are arranged opposite to each other. When the first connecting shaft 110 and the first base 210 are coupled, the first component and the second component are aligned to form the first preset channel 300 in the closed state. After the first connecting shaft 110 and the first base 210 are detached, the first component and the second component are separated from each other, dividing the first preset channel 300 in its circumferential direction into two non-closed parts.

In some embodiments, the first connecting shaft 110 includes a first contact surface 111. The first base 210 includes a second contact surface 211 for contacting at least partially the first contact surface 111 when the first connecting shaft 110 and the first base 210 are coupled. The first preset channel 300 is arranged between the first contact surface 111 and the second contact surface 211.

In this way, when the first connecting shaft 110 and the first base 210 are coupled, the first contact surface 111 contacts at least partially the second contact surface 211, so that the first preset channel 300 arranged between the first contact surface 111 and the second contact surface 211 is in a closed state. After the first connecting shaft 110 and the first base 210 are detached, the first contact surface 111 is separated from the second contact surface 211, so that the first preset channel 300 arranged between the first contact surface 111 and the second contact surface 211 is in a non-closed state.

In some embodiments, the surface of the first contact surface 111 or the surface of the second contact surface 211 is provided with a first through slot 112. When the first base 210 is coupled to the first connecting shaft 110, the wall surface of the first through slot 112 (i.e., the first component) and a corresponding portion of the other contact surface (i.e., the second component) are engaged to form the first preset channel 300 in the closed state. When the first base 210 is detached from the first connecting shaft 110, the wall surface of the first through slot 112 is separated from the other contact surface, causing the first preset channel 300 to be in the non-closed state.

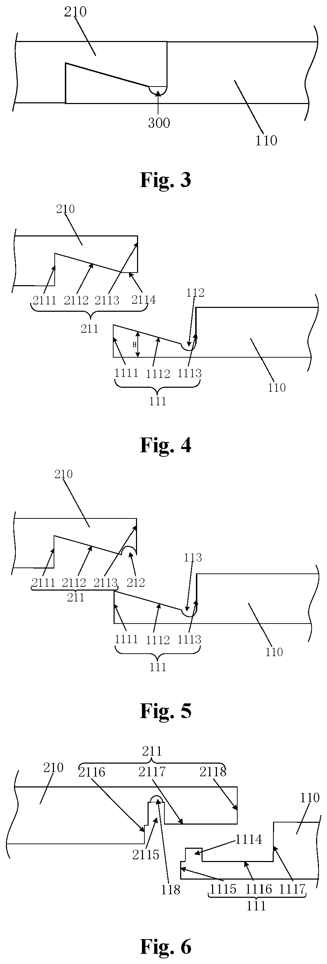

As shown in Fig. 4, the first through slot 112 is arranged on the first contact surface 111, and includes a first wall surface and a first opening. In some embodiments, a cross-section of the first wall surface is in the shape of an arc. For example, the cross-section of the first wall surface is in the shape of a circular arc. When the first connecting shaft 110 is coupled to the first base 210, the first opening fits a corresponding part of the second contact surface 211, the first wall surface is aligned with a corresponding part of the second contact surface 211, so that the first through slot 112 and the corresponding part of the second contact surface 211 jointly form the first preset channel 300 in the closed state. When the first connecting shaft 110 is detached from the first base 210, the first opening no longer fits the corresponding part of the second contact surface 211, and the first wall surface is separated from the corresponding part of the second contact surface 211, causing the first preset channel 300 to be in the non-closed state.

In another embodiment, as shown in Fig. 5, the surface of the first contact surface 111 is provided with a second through slot 113, and the surface of the second contact surface 211 is provided with a third through slot 212. When the first base 210 is coupled to the first connecting shaft 110, the wall surface of the second through slot 113 (i.e., the first component) and the wall surface of the third through slot 212 (i.e., the second component) jointly form the first preset channel 300 in the closed state. When the first base 210 is detached from the first connecting shaft 110, the wall surface of the second through slot 113 is separated from the wall surface of the third through slot 212, causing the first preset channel 300 to be in the non-closed state.

As shown in Fig. 5, the second through slot 113 includes a second wall surface and a second opening. The third through slot 212 includes a third wall surface and a third opening. In some embodiments, cross-sections of the second wall surface and the third wall surface are both in the shape of an arc. For example, the cross-sections of the second wall surface and the third wall surface are in the shape of a circular arc. When the first connecting shaft 110 is coupled to the first base 210, the second wall surface is aligned with the third wall surface, and the second opening fits the third opening, so that the second through slot 113 and the third through slot 212 jointly form the first preset channel 300 in the closed state. When the first connecting shaft 110 is detached from the first base 210, the second wall surface and the third wall surface are separated from each other, and the first opening no longer fits the second slot opening, causing the first preset channel 300 to be in the non-closed state.

It should be further noted that the side wall of any of the aforementioned through slots includes a notch which is connected to the opening of the through slot and configured to receive a part of the first control wire 120. After the first preset channel 300 is formed by the above through slot, the notch of the side wall of the through slot forms an end opening of the first preset channel 300.

It should be further noted that in the embodiment, when any of the contact surfaces mentioned above are in contact to form the first preset channel 300 in the closed state, at least the contact surfaces near the through slots forming the first preset channel 300 fit each other to prevent the first control wire 120 from sliding out of the first preset channel 300. At the same time, the two through slots cannot fit each other or the through slot and a corresponding part of another contact surface cannot fit each other, otherwise the first preset channel 300 in the closed state cannot be formed.

In some embodiments, the first contact surface 111 and the second contact surface 211 are configured such that a contact portion between the second contact surface 211 and the first contact surface 111 forms a constraint such that when the first connecting shaft 110 moves away from the first base 210 along the axis of the first connecting shaft 110, the first connecting shaft 110 is driven to move relative to the first base 210 along a radial direction of the first connecting shaft. In this way, constraining the first connecting shaft 110 from moving relative to the first base 210 along its axial direction can be converted into constraining the first connecting shaft 110 from moving relative to the first base 210 along its radial direction, which facilitates the coupling between the first connecting shaft 110 and the first base 210.

As shown in Figs. 4 and 5, the first contact surface 111 includes a first distal blocking surface 1111 at a distal end, a first proximal blocking surface 1113 at a proximal end, and a first inclined surface 1112 arranged between the first distal blocking surface 1111 and the first proximal blocking surface 1113. The second contact surface 211 includes a second distal blocking surface 2111 at a distal end, a second proximal blocking surface 2113 at a proximal end, and a second inclined surface 2112 arranged between the second distal blocking surface 2111 and the second proximal blocking surface 2113. The second distal blocking surface 2111 is configured to abut against the first distal blocking surface 1111. The second proximal blocking surface 2113 is configured to abut against the first proximal blocking surface 1113. The first inclined surface 1112 is configured to be at least partially aligned with the second inclined surface 2112. The maximum vertical distance (H, as shown in Fig. 4) from the edge of the first connecting shaft 110 to the first inclined surface 1112 decreases as the distance to the distal end of the first base 210 increases. Similarly, the maximum vertical distance from the edge of the first base 210 to the second inclined surface 2112 decreases as the axial distance from the second inclined surface 2112 to the second proximal blocking surface 2113 increases. Here, the maximum vertical distance from the edge of the first connecting shaft 110 to the first inclined surface 1112 refers to the greatest vertical distance among all vertical distances between each point on the edge of the first connecting shaft 110 and the intersection line formed by the first inclined surface 1112 and the cross-section in the same cross-section. The maximum vertical distance from the edge of the first base 210 to the second inclined surface 2112 refers to the greatest vertical distance among all vertical distances between each point on the edge of the first base 210 and the intersection line formed by the second inclined surface 2112 and the cross-section in the same cross-section.

By providing the first inclined surface 1112, a contact portion between the second contact surface 211 and the first contact surface 111 allows the first connecting shaft 110 to move relative to the first base 210 in the radial direction of the first connecting shaft 110 when the first connecting shaft 110 moves away from the first base 210 in the axial direction of the first connecting shaft 110.

In some embodiments, the first inclined surface 1112 is an inclined plane, a curved surface, or a wavy surface. For example, the first inclined surface 1112 is an inclined plane or a curved surface. Thus, the manufacture of the first inclined surface 1112 can be facilitated.

In some embodiments, the first through slot 112 is arranged on the first inclined surface 1112 or the second inclined surface 2112.

In one exemplary embodiment, as shown in Fig. 4, the first through slot 112 is arranged on the first inclined surface 1112, and the second contact surface 211 may further include a transition surface 2114 arranged between the second inclined surface 2112 and the second proximal blocking surface 2113.For example, the transition surface 2114 is parallel to the axis of the first connecting shaft 110. The first through slot 112 is arranged on the first inclined surface 1112 adjacent to the first proximal blocking surface 1113 and corresponds to the transition surface 2114. When the first connecting shaft 110 is coupled to the first base 210, the first distal blocking surface 1111 fits the second distal blocking surface 2111, the first inclined surface 1112 fits the second inclined surface 2112, the first proximal blocking surface 1113 fits the second proximal blocking surface 2113, and the first through slot 112 and the transition surface 2114 form the first preset channel 300 in the closed state. The first through slot 112 includes a first wall surface and a first opening. When the first connecting shaft 110 is coupled to the first base 210, the first opening fits the transition surface 2114, and the first wall surface of the first through slot 112 is aligned with the transition surface 2114, forming the side wall of the first preset channel 300 in the closed state. After the first connecting shaft 110 is detached from the first base 210, the first inclined surface 1112 is separated from the second inclined surface 2112, so that the first opening of the first through slot 112 no longer fits the transition surface 2114, and the first wall surface of the first through slot 112 forming the side wall of the first preset channel 300 is separated from the transition surface 2114. Thus, the first preset channel 300 is no longer closed. In some embodiments, the first through slot 112 may also be disposed at other positions on the first contact surface 111 or the second contact surface 211.

For example, the first through slot 112 is arranged on the second inclined surface 2112, the first contact surface 111 may include a transition surface between the first inclined surface 1112 and the first proximal blocking surface 1113, and the second contact surface 211 is no longer provided with the transition surface 2114. Similarly, when the first connecting shaft 110 is coupled to the first base 210, the first through slot 112 and the transition surface of the first contact surface 111 jointly form the first preset channel 300 in the closed state. After the first connecting shaft 110 is detached from the first base 210, the first through slot 112 is separated from the transition surface of the first contact surface 111, and the first preset channel 300 is no longer closed.

In another exemplary example, as shown in Fig. 5, the second through slot 113 is arranged on the surface of the first contact surface 111 and is adjacent to the first proximal blocking surface 1113. The third through slot 212 is arranged on the surface of the second contact surface 211 and is adjacent to the second proximal blocking surface 2113. When the first connecting shaft 110 is coupled to the first base 210, the first distal blocking surface 1111abuts against the second distal blocking surface 2111, the first inclined surface 1112 abuts against the second inclined surface 2112, the first proximal blocking surface 1113 abuts against the second proximal blocking surface 2113, and the second through slot 113 and the third through slot 212 form the first preset channel 300 in the closed state. After the first connecting shaft 110 is detached from the first base 210, the first inclined surface 1112 is separated from the second inclined surface 2112, so that the second through slot 113 is separated from the third through slot 212. Thus, the first preset channel 300 is no longer closed.

In some embodiments, the delivery device 100 further includes a first central rod 130. The first central rod 130 is configured such that when the first central rod 130 is positioned at the coupling site between the first connecting shaft 110 and the first base 210, the first connecting shaft 110 remains stationary at least in the radial direction relative to the first base 210, thereby achieving the coupling between the first connecting shaft 110 and the first base 210. Thus, when combined with the above embodiments in which a contact portion between the second contact surface 211 and the first contact surface 111 constrains the first connecting shaft 110 from moving relative to the first base 210 in the radial direction of the first connecting shaft 110 when the first connecting shaft 110 moves away from the first base 210 in the axial direction of the first connecting shaft 110, the coupling between the first connecting shaft 110 and the first base 210 can be achieved by using the first central rod 130 to remain the first connecting shaft 110 stationary at least in the radial direction relative to the first base 210.

In one example, a receiving channel is disposed on the first connecting shaft 110 and the first base 210 along the axial direction of the first connecting shaft 110. When the first central rod 130 is placed into the receiving channel and positioned at the coupling site between the first connecting shaft 110 and the first base 210, the first connecting shaft 110 remains stationary at least in the radial direction relative to the first base 210.

The receiving channel includes a first receiving channel arranged on the first connecting shaft 110 along the axial direction of the first connecting shaft 110. The receiving channel further includes a second receiving channel arranged on the first base 210 and coaxial with the first receiving channel. The first central rod 130 passes through the first receiving channel and extends to the coupling site between the first connecting shaft 110 and the first base 210, so that the first connecting shaft 110 remains stationary at least in the radial direction relative to the first base 210. When the first connecting shaft 110 is coupled to the first base 210, the first receiving channel and the second receiving channel connect each other and jointly form the above receiving channel.

In another example, the first central rod 130 is a hollow rod, and when the first central rod 130 is sleeved at the coupling site between the first connecting shaft 110 and the first base 210, the first connecting shaft 110 remains stationary at least in the radial direction relative to the first base 210. The first central rod 130 is also provided with an axial slot extending axially from the distal end toward the proximal end of the first central rod 130. The axial slot is aligned with the first preset channel 300 in the circumferential direction of the first connecting shaft 110, so that the connection part of the first segment 121 with the second segment 122 can pass through the axial slot and be positioned at or adjacent to the first preset channel 300. In this way, when the first central rod 130 moves, especially when the first central rod 130 is retracted toward the proximal end, the first segment 121 moves relative to the axial channel, avoiding interference between the first central rod 130 and the first segment 121. Such interference may cause changes in the position of the first control wire 120, subsequently affecting the posture and/or position of the first movable member.

In some embodiments, the connection between the first connecting shaft 110 and the first base 210 can achieve axial relative rest between the first connecting shaft 110 and the first base 210, but additional member is required to achieve radial relative rest between the first connecting shaft 110 and the first base 210. In another exemplary example, one of the first contact surface 111 and the second contact surface 211 is provided with a second protrusion 1114, and the other is provided with a second groove 2115. The protruding direction of the second protrusion 1114 and the recessing direction of the second groove 2115 are both in the radial direction of the connecting shaft 110. When the first contact surface 111 is at least partially in contact with the second contact surface 211, the second protrusion 1114 is fitted into the second groove 2115. In this way, when the second protrusion 1114 is fitted into the second groove 2115, if it is required to move the first connecting shaft 110 relative to the first base 210 along the axial direction of the first connecting shaft 110, it is necessary to first disengage the second protrusion 1114 from the second groove 2115. Further, since the protruding direction of the second protrusion 1114 and the recessing direction of the second groove 2115 are both in the radial direction of the first connecting shaft 110, disengaging the second protrusion 1114 from the second groove 2115 requires to move the first connecting shaft 110 relative to the first base 210 in the radial direction of the first connecting shaft 110. In this way, it can be achieved that when the first connecting shaft 110 moves away from the first base 210 along the axial direction of the first connecting shaft 110, the first connecting shaft 110 needs to first move relative to the first base 210 in the radial direction of the first connecting shaft 110, thereby facilitating the coupling between the first connecting shaft 110 and the first base 210. In this embodiment, similar to the above embodiments, there may be two through slots forming the first preset channel 300, which are respectively arranged on the first contact surface 111 and the second contact surface 211. There may be only one through slot constituting the first preset channel 300, which is arranged on the first contact surface 111 or the second contact surface 211. The through slot may be arranged on the second protrusion 1114, or on the slot wall or slot bottom of the second groove 2115. Of course, the through slot may be positioned on other locations of the first contact surface 111 and/or the second contact surface 211.

As shown in Fig. 6, there is only one through slot forming the first preset channel 300, that is eighth through slot 118. In some embodiments, the eighth through slot 118 is arranged at the slot bottom of the second groove 2115.

In this example, the first contact surface 111 may include a third distal blocking surface 1115 at the distal end, a first connection surface 1116 extending from the proximal end toward the distal end, and a third proximal blocking surface 1117 at the proximal end. The second contact surface 211 may include a fourth distal blocking surface 2116 at the distal end for fitting the third distal blocking surface 1115, a second connection surface 2117 extending from the proximal end toward the distal end for fitting the first connection surface 1116, and a fourth proximal blocking surface 2118 at the proximal end for fitting the third proximal blocking surface 1117. In this case, one of the second protrusion 1114 and the second groove 2115 may be arranged on the first connection surface 1116, and the other may be arranged on the second connection surface 2117. In this case, the first connection surface 1116 and the second connection surface 2117 may be parallel to the axis of the first connecting shaft 110. The first connecting shaft 110 and the first base 210 are rod-shaped, and the second protrusion 1114 and the second groove 2115 are both circular rings with notches.

In this case, the delivery device 100 may further be provided with a first central rod 130. The first central rod 130 may be configured such that when the first central rod 130 is positioned at the coupling site between the first connecting shaft 110 and the first base 210, the first connecting shaft 110 remains stationary at least in the radial direction relative to the first base 210, to achieve the coupling between the first connecting shaft 110 and the first base 210. For example, a receiving channel is disposed on the first connecting shaft 110 and the first base 210 along the axial direction of the first connecting shaft 110. The protrusion length of the second protrusion 1114 is long enough, so that the receiving channel partially overlaps with the eighth through slot 118. When the first central rod 130 is placed into the receiving channel and positioned at the coupling site between the first connecting shaft 110 and the first base 210, the first connecting shaft 110 remains stationary at least in the radial direction relative to the first base 210, thereby achieving a fixed connection between the first connecting shaft 110 and the first base 210. At the same time, at least part of the wall surface of the eighth through slot 118 and the wall surface of the first central rod 130 jointly form the preset channel 300 in the closed state.

In some embodiments, the first connecting shaft 110 is provided with a first coupling portion 114, and the first base 210 is provided with a second coupling portion 213. The first coupling portion 114 or the second coupling portion 213 is biased from the axial direction of the first connecting shaft 110. When the biased coupling portions are driven to be arranged along the axial direction of the first connecting shaft 110, the first coupling portion 114 and the second coupling portion 213 are coupled, with the first contact surface 111 arranged on a side of the first coupling portion 114 facing the second coupling portion 213, and the second contact surface 211 arranged on a side of the second coupling portion 213 facing the first coupling portion 114. In this embodiment, as shown in the above embodiments, there may be two through slots forming the first preset channel 300, which are respectively arranged on the first contact surface 111 and the second contact surface 211. There may be only one through slot forming the first preset channel 300, which is arranged on the first contact surface 111 or the second contact surface 211.

In an embodiment, one of the first coupling portion 114 and the second coupling portion 213 is provided with a first protrusion 115, and the other is provided with a first groove 214. When the first coupling portion 114 or the second coupling portion 213 is biased from the axial direction of the first connecting shaft 110, the first protrusion 115 is disengaged from the first groove 214. When the biased coupling portions are driven to be arranged along the axial direction of the first connecting shaft 110, the first protrusion 115 is received in the first groove 214.

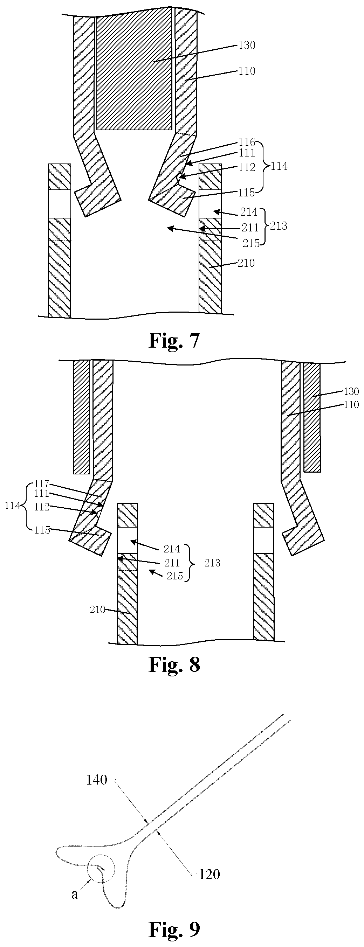

Referring to Fig. 7, the first coupling portion 114 further includes an inwardly biased first support 116. The first protrusion 115 is arranged on the distal end of the first support 116 and extends perpendicular to the axial direction of the first support 116. The first base 210 is provided with a second receiving channel 215 for receiving the first coupling portion 114, and the first groove 214 is arranged on the wall surface of the second receiving channel 215. When the first support 116 is driven to deflect so that the axis of the first support 116 is parallel to the axis of the first connecting shaft 110, the first protrusion 115 is received in the first groove 214. In this case, the first contact surface 111 may be a surface of the first support 116 facing the wall surface of the second receiving channel 215, and the second contact surface 211 may be a part of the wall surface of the second receiving channel 215 facing the first support 116. As described in the above embodiments, the surface of the first contact surface 111 or the surface of the second contact surface 211 is provided with the first through slot 112; or, the surface of the first contact surface 111 is provided with the second through slot 113, and the surface of the second contact surface 211 is provided with the third through slot 212, which are not further described here.

Referring to Fig. 8, the first coupling portion 114 further includes an outwardly biased support 117. The first protrusion 115 is arranged on the distal end of the support 117 and protrudes inwardly perpendicular to the axial direction of the support 117. The first groove 214 is arranged on the outer wall surface of the first base 210. When the support 117 is driven to deflect so that the axis of the support 117 is parallel to the axis of the connecting shaft 110, the first protrusion 115 is received in the first groove 214. In this case, the first contact surface 111 may be a surface of the support 117 facing the outer wall surface of the first base 210, and the second contact surface 211 may be a part of the outer wall surface of the first base 210 facing the support 117. As described in the above embodiments, the surface of the first contact surface 111 or the surface of the second contact surface 211 is provided with the first through slot 112; or, the surface of the first contact surface 111 is provided with the second through slot 113, and the surface of the second contact surface 211 is provided with the third through slot 212, which are not further described here.

In some embodiments the delivery device 100 further includes a first central rod 130. The first central rod 130 is configured such that when the first central rod 130 is positioned at the coupling site between the first connecting shaft 110 and the first base 210, the first central rod 130 drives the biased coupling portion to be arranged along the axial direction of the first connecting shaft 110.

Referring to Fig. 7, a receiving channel is disposed on the first connecting shaft 110 and the first base 210 along the axial direction of the first connecting shaft 110. When the first central rod 130 is placed in the receiving channel and positioned at the coupling site between the first connecting shaft 110 and the first base 210, the biased coupling portion is driven to be arranged along the axial direction of the first connecting shaft 110. In this example, the first coupling portion 114 includes the inwardly biased first support 116 mentioned in the above embodiments. After the first central rod 130 is placed into the receiving channel, the first central rod 130 moves toward the distal end and passes through the first coupling portion 114 and the second coupling portion 213 in order to abut against the first support 116, and drive the first support 116 to deflect outwardly. When the axis of the first support 116 is parallel to the axis of the first connecting shaft 110, the first protrusion 115 is received in the first groove 214, thus achieving the fixation of the first connecting shaft 110 to the first base 210 (i.e., coupling between the first connecting shaft 110 and the first base 210). On the other hand, the first through slot 112, which is arranged on the surface of the first contact surface 111 or the surface of the second contact surface 211, forms the preset channel 300 in the closed state together with the other contact surface; or, the second through slot 113 arranged on the surface of the first contact surface 111 forms the preset channel 300 in the closed state together with the third through slot 212 arranged on the surface of the second contact surface 211.

Referring to Fig. 8, the first central rod 130 is a hollow rod. When the first central rod 130 is sleeved at the coupling site between the first connecting shaft 110 and the first base 210, the biased coupling portion is driven to be arranged along the axial direction of the first connecting shaft 110. The first coupling portion 114 includes the above-mentioned outwardly biased support 117. After the first central rod 130 is sleeved at the coupling site between the first connecting shaft 110 and the first base 210, the first central rod 130 is sleeved outside the first coupling portion 114 and the second coupling portion 213 in order to abut against the support 117 and drive the support 117 to deflect inwardly. When the axis of the support 117 is parallel to the axis of the first connecting shaft 110, the first protrusion 115 is received in the first groove 214, thus achieving the fixation of the first connecting shaft 110 to the first base 210. On the other hand, the first through slot 112, which is arranged on the surface of the first contact surface 111 or the surface of the second contact surface 211, forms the preset channel 300 in the closed state together with the other contact surface; or, the second through slot 113 arranged on the surface of the first contact surface 111 forms the preset channel 300 in the closed state together with the third through slot 212 arranged on the surface of the second contact surface 211. In some embodiments, the first central rod 130 may also be provided with the configuration that an axial slot extends axially from the distal end toward the proximal end of the first central rod 130, and the receiving channel is arranged to correspond to the first preset channel 300 in the circumferential direction of the first connecting shaft 110, which has the similar structure and function as in the other examples described above. This is not further described here.

In some embodiments, the first preset channel 300 includes a first portion, a second portion, and a channel middle portion. The portion of the first preset channel 300 which is close to the first segment 121 of the first control wire 120 and distant from the second segment 122 of the first control wire 120 is defined as the first portion, the portion of the first preset channel 300 which is distant from the first segment 121 of the first control wire 120 and close to the second segment 122 of the first control wire 120 is defined as the second portion, and the portion of the first preset channel 300 arranged between the first portion and the second portion is defined as the channel middle portion.

In some embodiments, the diameter of the second segment 122 is larger than the diameter of the first segment 121. In this way, it can be easily achieved that the second segment 122 is constrained within the first preset channel 300 in the closed state when the first preset channel 300 is in the closed state, so that the distal end of the first segment 121 is constrained at the coupling site. In some embodiments, the diameter of the second segment 122 is smaller than the diameter of the channel middle portion, and the diameter of the second segment 122 is larger than the diameter of the first portion. In this way, the second segment 122 cannot escape from the first preset channel 300 in the direction from the channel middle portion toward the first portion, and is constrained within the first preset channel 300. In some embodiments, the diameter of the second segment 122 is larger the diameter of the second portion. In this way, the second segment 122 cannot escape from the first preset channel 300 in the direction from the channel middle portion toward the second portion, which further ensures that the second segment 122 is constrained within the first preset channel 300 and cannot escape.

**In** some embodiments, the first control wire 120 further includes a third segment 123 connected to the end of the second segment 122 away from the first segment 121. The diameter of the second segment 122 is larger than the diameter of each of the first segment 121 and the third segment 123, or the diameter of the second segment 122 is smaller than the diameter of the portion of each of the first segment 121 and the third segment 123 that is connected to the second segment 122. In this way, it can be easily achieved that the second segment 122 is constrained within the first preset channel 300 in the closed state when the first preset channel 300 is in the closed state, so that the distal ends of the first segment 121 and the third segment 123 are constrained at the coupling site.

**In** some embodiments, a radial dimension of the portion of each of the first segment 121 and the third segment 123 that is connected to the second segment 122 is larger than a radial dimension of the second segment 122, a minimum radial dimension of the first preset channel 300 in the closed state is larger than or equal to the radial dimension of the second segment 122, and a maximum radial dimension of the first preset channel 300 in the closed state is smaller than the radial dimension of the portion of each of the first segment 121 and the third segment 123 that is connected to the second segment 122. In this way, it can be achieved that when the first preset channel 300 is in the closed state, the portion of each of the first segment 121 and the third segment 123 that is connected to the second segment 122 cannot be placed inside the first preset channel 300, and the second segment 122 can be constrained within the first preset channel 300.

Alternatively, a radial dimension of each of the first segment 121 and the third segment 123 is smaller than a radial dimension of the second segment 122, a maximum radial dimension of the first preset channel 300 in the closed state is larger than or equal to the radial dimension of the second segment 122, a minimum radial dimension of the first preset channel 300 in the closed state is smaller than or equal to the radial dimension of the second segment 122, and the maximum radial dimension of the first preset channel 300 in the closed state is not equal to the minimum radial dimension. For example, the diameter of the second segment 122 is smaller than the diameter of the channel middle portion, and the diameter of the second segment 122 is larger than the diameter of each of the first portion and the second portion. In this way, the second segment 122 is constrained within the first preset channel 300 in the closed state and cannot escape from the first preset channel 300, and each of the first segment 121 and the third segment 123 is constrained at the coupling site, so that the first segment 121 can control the movement of the first movable member.

In some embodiments, the prosthesis further includes a second movable member. The third segment 123 is configured to independently drive the second movable member to move. When the first connecting shaft 110 and the first base 210 are coupled, the first preset channel 300 is in a closed state, and the second segment 122 is constrained within the first preset channel 300 in the closed state, thereby constraining the first segment 121 and the third segment 123 at the coupling site. In this way, using one control wire (i.e., the first control wire 120 in this embodiment) can control the movement of two movable members (i.e., the first movable member and the second movable member).

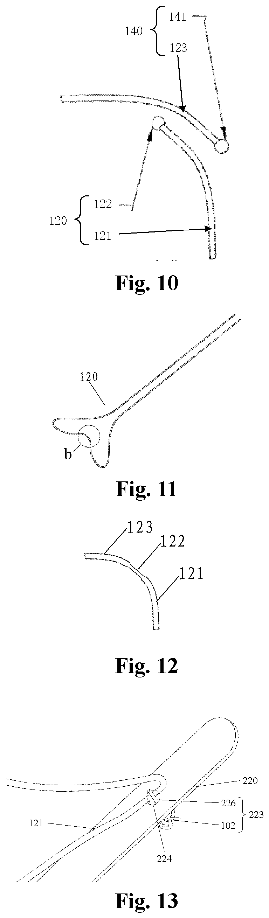

Referring to Fig. 11 and Fig. 12, this embodiment differs from the above embodiments in that the third segment 123 is further configured to control the second movable member. The third segment 123 extends to the proximal end of the delivery device 100, so that the third segment 123 can be manipulated at the proximal end of the delivery device 100 to control the second movable member. The specific structure and dimension of the third segment 123 are not further described here.

Referring to Fig. 9 and Fig. 10, in some embodiments, the prosthesis also includes a second movable member. The delivery device 100 further includes a second control wire 140. The second control wire 140 is provided with a third segment 123 for independently driving movement of the second movable member and a fourth segment 141, which are connected in sequence. When the first preset channel 300 is in the closed state, the fourth segment 141 is constrained within the first preset channel 300, thereby constraining the third segment 123 at the coupling site. When the first preset channel 300 is in the non-closed state, the second control wire 120 can be detached from the first preset channel 300. In this way, using the first control wire 120 can control the movement of the first movable member, and using the second control wire 140 can control the movement of the second movable member.

In some embodiments, the second portion is defined as the portion that is close to the third segment 123 and distant from the fourth segment 141. The radial dimension of the second segment 122 is larger than the radial dimension of the first segment 121 and larger than the radial dimension of the first portion of the first preset channel 300 in the closed state. The radial dimension of the fourth segment 141 is larger than the radial dimension of the third segment 123 and larger than the radial dimension of the second portion of the first preset channel 300 in the closed state. In this way, when the second segment 122 and the fourth segment 141 are constrained within the first preset channel 300 in the closed state, it can be ensured that the second segment 122 cannot escape from the first portion and the fourth segment 141 cannot escape from the second portion, so that the first control wire 120 can be used to control the movement of the first movable member and the second control wire 140 can be used to control the movement of the second movable member. In some embodiments, the dimension of the channel middle portion of the first preset channel 300 in the closed state is larger than the radial dimension of the second segment 122 and larger than the radial dimension of the fourth segment 141, thereby ensuring that the second segment 122 and the fourth segment 141 can be received in the first preset channel 300 in the closed state. In some embodiments, the diameter of the second segment 122 is larger than the diameter of the second portion, and the diameter of the fourth segment 141 is larger than the diameter of the first portion. In this way, the second segment 122 cannot escape from the second portion, and the fourth segment 141 cannot escape from the first portion, thereby further ensuring that the second segment 122 and the fourth segment 141 are constrained within the first preset channel 300 in the closed state.

With further reference to Figs. 1-3 and Figs. 13-15, in some embodiments, the prosthesis includes one movable member, namely the first movable member. Controlling the movement of the first movable member via the first segment 121 can change state of the prosthesis. The "change of the state" here may refer to the changes in the structure, size, and shape of the entire or part of prosthesis. For example, the prosthesis changes from a compressed state to an expanded state, such as a covered stent used to treat aortic stenosis. A restraint wire (i.e., the first movable member) is typically used during delivery to restrain the covered stent in a compressed state. Once the covered stent is positioned at the target location, the restraint wire is separated from the covered stent via the control wire, allowing the covered stent to transition from a compressed state to an expanded state. The "change of state" here may also refer to the implementation of the unlocked state/locked state between the prosthesis and the target tissue, between the prosthesis and the delivery system, and between the internal components of the prosthesis, and/or the mutual transformation between the locked state and the unlocked state. For example, when delivering the left atrial appendage occluder, the control wire and the left atrial appendage occluder are in a locked state. After the left atrial appendage occluder is released, the control wire is driven to be detached from the left atrial appendage occluder, allowing the left atrial appendage occluder to be in an unlocked state. The "change of state" may also refer to changes in the position and/or posture of the entire or part of the prosthesis (such as the first movable member). For example, in the case of the mitral valve clip used to treat mitral valve regurgitation, the control wire is used to control the relative rotation of the gripper (i.e., the first movable member) in the mitral valve clip, thereby achieving the clamping of the mitral valve leaflets.

In some embodiments, the prosthesis includes two movable members, namely a first movable member and a second movable member. State change of the prosthesis is achieved by controlling the movement of the first movable member via the first segment 121 and controlling the movement of the second movable member via the third segment 123. When it is required to detach the delivery device 100 from the prosthesis, the first preset channel 300 can be operated to be in the non-closed state, so that the first control wire 120 (or the first control wire 120 and the second control wire 140) can be disengaged from the first preset channel 300, facilitating the withdrawal of the first control wire 120 (or the first control wire 120 and the second control wire 140) from the human body and detaching the first control wire 120 (or the first control wire 120 and the second control wire 140) from the prosthesis.

This embodiment does not limit the specific type of prosthesis. In some embodiments, the prosthesis is a mitral valve clip 200 for treating mitral valve regurgitation. In some embodiments, the prosthesis is a tricuspid valve clip used to treat tricuspid valve regurgitation. In some embodiments, the prosthesis is a stent-graft for treating aortic stenosis. In some embodiments, the prosthesis is a left atrial appendage occluder or an intracranial coil. In the following, the prosthesis is a mitral valve clip 200 by way of example. It should be emphasized that the prosthesis being a mitral valve clip 200 is only an example and does not constitute a limitation on the present disclosure.

The mitral valve clip 200 includes a first base 210 and a gripper (i.e., movable member) rotatable relative to the axis of the first base 210.The mitral valve clip 200 further includes an arm rotatable relative to the axis of the first base 210. The gripper is arranged on the proximal end of the first base 210, and the arm is arranged on the distal end of the first base 210. The clamping of the native valve leaflet edge of the mitral valve is achieved by rotation of the gripper and the arm. In some embodiments, the gripper includes a first gripper 220 (i.e., first movable member) and a second gripper 230 (i.e., second movable member). And the first gripper 220 and the second gripper 230 are symmetrically arranged about the axis of the first base 210.

It still needs to be emphasized that the first movable member here being the first gripper 220 and the second movable member being the second gripper 230 is only an example when the prosthesis is a mitral valve clip 200. In some embodiments, the first movable member may not be the first gripper 220, and the second movable member may not be the second gripper 230. The specific types of the first movable member and the second movable member are not limited in the present disclosure.

Similarly, the arm includes a first arm 240 and a second arm 250, which are symmetrically arranged about the axis of the first base 210 and arranged on the plane which the first gripper 220 and the second gripper 230 are defined, to better clamp the edges of the anterior leaflet and posterior leaflet of the mitral valve. Here, the plane defined by the first gripper 220 and the second gripper 230 refers to the plane formed by the axis of the first gripper 220 and the axis of the second gripper 230; the first arm 240 and the second arm 250 being arranged on the plane which the first gripper 220 and the second gripper 230 are defined refers to that the axes of the first arm 240 and the second arm 250 are both arranged on the plane defined by the first gripper 220 and the second gripper 230. After the mitral valve clip 200 is delivered to the target position of the mitral valve, the gripper and the arm are driven to rotate to clamp the anterior leaflet and posterior leaflet of the mitral valve by the gripper and the arm, achieving the treatment of mitral valve regurgitation.

In some embodiments, the first gripper 220 and the second gripper 230 are elastically connected via a gripper transition portion 225. The gripper transition portion 225 is arranged at the distal end of the first base 210. The first gripper 220 and the second gripper 230 are both in the form of a sheet. In an embodiment, the first gripper 220, the gripper transition portion 225, and the second gripper 230 are integrally molded to form a structure similar to an inverted omega shape, and the first gripper 220 and the second gripper 230 are inherently in an extended state. As shown in Fig. 2, when the first segment 121 is pulled, the first gripper 220 is driven to rotate to move upward and closer to the first base 210, and the first gripper 220 undergoes elastic deformation. When the first segment 121 is no longer pulled, the first gripper 220 rotates away from the first base 210 to a desired position. Similarly, when the third segment 123 is pulled, the second gripper 230 is driven to rotate to move upward and closer to the first base 210, and the second gripper 230 undergoes elastic deformation. When the third segment 123 is no longer pulled, the second gripper 230 rotates away from the first base 210 to a desired position.

The first arm 240 and the second arm 250 are both rotatably connected to the first base 210. In some embodiments, the first arm 240 and the second arm 250 can be both rotatably connected to the first base 210 via a same rotating shaft. In some embodiments, the cross-sectional shape of the first arm 240 perpendicular to the axial direction is U-shaped to better wrap the first leaflet edge 401 of the mitral valve together with the first gripper 220. Similarly, the cross-sectional shape of the second arm 250 perpendicular to the axial direction is U-shaped to better wrap the second leaflet edge 402 of the mitral valve together with the second gripper 230. When the mitral valve clip 200 clamps the mitral valve leaflets, the first arm 240 and the second arm 250 are driven to rotate, and compress the edges of the mitral valve leaflets respectively with the first gripper 220 and the second gripper 230, so as to achieve the clamping of the mitral valve leaflets.

There are no specific limitations on the manner by which the first segment 121 controls the movement of the first movable member, or even on the method by which the third segment 123 controls the movement of the second movable member. For the mitral valve clip 200, it only requires that the first segment 121 is movably connected to the first gripper 220. Thus, on the one hand, the first gripper 220 is rotatable along with the movement of the first segment 121; and on the other hand, when the delivery device 200 is required to be separated from the mitral valve clip 200, the first control wire 120 can be detached from the first gripper 220. The relationship between the third segment 123 and the second gripper 230 is also the same, and is not further described.

Referring to Figs. 9, 11, and 13 to 15, the first segment 121 extends distally from the first preset channel 300, and after extending to the first gripper 220, it extends back towards the proximal end of the delivery device 200. In this way, the first segment 121 forms a curved portion at the position near the first gripper 220, and the curved portion interpenetrates with the first gripper 220 or with an attachment arranged on the first gripper 220, so as to control the rotation of the first gripper 220.

In some embodiments, the second segment 122 is configured to be detachable from the first gripper 220. In this way, when it is required to separate the first control wire 120 from the first gripper 220, it can be ensured that the second segment 122 can be detached from the first gripper 220.

If the third segment 123 is part of the first control wire 120, the third segment 123 is also configured to be detachable from the first gripper 220. In this way, when it is required to detach the first control wire 120 from the first gripper 220, it can be ensured that the third segment 123 can be detached from the first gripper 220.

If the third segment 123 is part of the second control wire 140, the structure between the third segment 123 and the second gripper 230 is the similar as the structure between the first segment 121 of the first control wire 120 and the first gripper 220 described above, and is not further described here. The structure between the fourth segment 141 and the second gripper 230 is the similar as the structure between the second segment 122 of the first control wire 120 and the first gripper 220 described above, and is not further described here.

In some embodiments, if the third segment 123 is a part of the first control wire 120, the connection portion between the third segment 123 and the second segment 122 is a first transition portion, and the cross-sectional area of the first transition portion gradually increases in the direction from the second segment 122 towards the third segment 123. In this way, there is no obvious discontinuity at the connection portion between the second segment 122 and the third segment 123. During the process of withdrawing the first control wire 120, the entire first control wire 120 needs to be detached from the first gripper 220. In this way, the second segment 122 and the third segment 123 can smoothly and sequentially detach from the first gripper 220, avoiding any impact on the posture and/or position of the first gripper 220. Moreover, by providing a smooth first transition portion, when withdrawing the first control wire 120, the first control wire 120 can be avoided from getting stuck on another component of the prosthetic system, and can also be avoided from wearing out, thereby reducing the possibility of fracture of the first control wire 120.

Similarly, if the third segment 123 is part of the first control wire 120, the connection portion between the first segment 121 and the second segment 122 may be a second transition portion that has the similar function as the first transition portion, which is not further described here.

Referring to Fig. 13, the first gripper 220 is provided with an attachment, which is a fixing wire 223. The fixing wire 223 is arranged on the first gripper 220 and forms a wire-body 226 for interpenetrating with the aforementioned curved portion.

In some embodiments, the wire-body 226 is annular to form a first through hole for interpenetrating with the aforementioned curved portion. The fixing wire 223 further includes a fixing knot 102 connected to the wire-body 226, and the fixing knot 102 is arranged on the first gripper 220. That is, the fixing wire 223 forms a structure similar to a knot. The curved portion of the first segment 121 passes through the first through hole to interpenetrate with the wire-body 226.

In some embodiments, the first gripper 220 is provided with a second through hole 224 on its surface, and the wire-body 226 passes through the second through hole 224 from the side of the first gripper 220 away from the first base 210. The fixing knot 102 is arranged on the side of the first gripper 220 away from the first base 210, and the fixing knot 102 is configured to be unable to pass through the second through hole 224.

Alternatively, the fixing knot 102 of the fixing wire 223 is wound around the second through hole 224 to be fixedly connected to the first gripper 220.

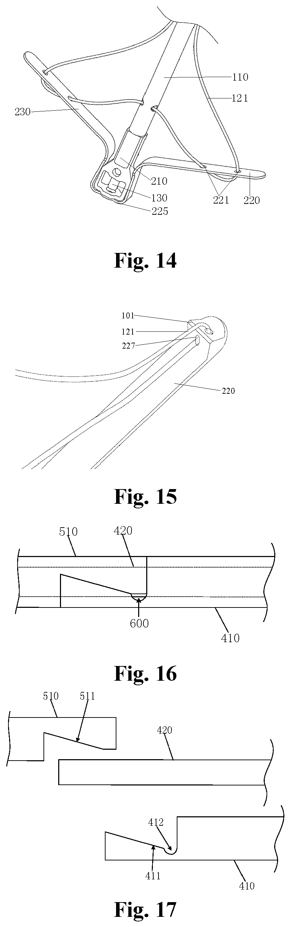

Referring to Fig. 14, the first gripper 220 is provided with two third through holes 221. The end of the first segment 121 away from the second segment 122 passes through one third through hole 221 from the side of the first gripper 220 away from the first arm 240, and then passes through the other third through hole 221 from the side of the first gripper 220 close to the first arm 240. In this way, the curved portion of the first segment 121 can interpenetrate with the first gripper 220.

Alternatively, the first gripper 220 is provided with an even number of third through holes 221, more than two in total, and the end of the first segment 121 away from the second segment 122 alternatingly passes through these third through holes 221. In this way, the curved portion of the first segment 121 can also interpenetrate with the first gripper 220.

Referring to Fig. 15, the first gripper 220 is provided with a third protrusion 101, and the third protrusion 101 on the first gripper 220 is provided with a fourth through hole 227. The end of the first segment 121 away from the second segment 122 passes through the fourth through hole 227. In this way, the curved portion of the first segment 121 can also interpenetrate with the first gripper 220. In some embodiments, an extension direction of the fourth through hole 227 is the same as an extension direction of the first gripper 220.

It should be noted that the connection manner between the third segment 123 and the second gripper 230 can refer to the connection manner between the first segment 121 and the first gripper 220 described above, and is not further described here.

If the third segment 123 is part of the first control wire 120 and the third segment 123 controls the rotation of the second gripper 230, the first segment 121 and the second segment 122 can both be configured to be detachable from the second gripper 230. In this way, when it is required to detach the first control wire 120 from the second gripper 230, it can be ensured that the first segment 121 and the second segment 122 can be detached from the second gripper 230.

In some embodiments, the mitral valve clip 200 further includes an actuating assembly configured to control the rotation of the first arm 240 and the second arm 250.

Referring to Figs. 1 and 2, the actuating assembly includes a first connecting rod 261, a second connecting rod 262, and a slider 263. The proximal end of the first connecting rod 261 is rotatably connected to the first arm 240, and the distal end thereof is rotatably connected to the slider 263. The slider 263 can move axially relative to the first base 210. Thus, the first connecting rod 261, the first arm 240, the first base 210, and the slider 263 form a kinematic mechanism similar to a crank-slider mechanism. When the slider 263 is driven to move close to or away from the first base 210, the first arm 240 is driven to rotate. Similarly, the proximal end of the second connecting rod 262 is rotatably connected to the second arm 250, and the distal end thereof is rotatably connected to the slider 263. Similarly, the second connecting rod 262, the second arm 250, the first base 210, and the slider 263 also form a kinematic mechanism similar to a crank-slider mechanism. When the slider 263 is driven to move close to or away from the first base 210, the second arm 250 is driven to rotate.

In some embodiments, the slider 263 is T-shaped, that is, the slider 263 includes a horizontal portion and a vertical portion. The first base 210 is of a hollow structure, and the vertical portion of the slider 263 is movably received within the first base 210. Two ends of the horizontal portion of the slider 263 are rotatably connected to the first connecting rod 261 and the second connecting rod 262 respectively. The vertical portion of the slider 263 is detachably connected to the first central rod 130, for example, through a threaded connection. In this way, the first central rod 130 can drive the slider 263 to move, to drive the first arm 240 and the second arm 250 to rotate. In some embodiments, the first connecting rod 261 and the second connecting rod 262 are symmetrically arranged about the axis of the first base 210. Thus, when the slider 263 drives the first arm 240 and the second arm 250 to move, the rotation angle of the first arm 240 is the same as the rotation angle of the second arm 250.

In some embodiments, the delivery device 100 further includes a catheter 150 sleeved on the first connecting shaft 110. The catheter 150 is provided with two second channels extending axially along the catheter 150. The end of the first segment 121 away from the second segment 122 passes through one second channel from the distal end of this second channel and extends to the proximal end of this second channel, and the end of the third segment 123 away from the second segment 122 (or the fourth segment 141) passes through the other second channel from the distal end of that second channel and extends to the proximal end of that second channel. In this way, it is convenient for the operator to pull the end of the first segment 121 away from the second segment 122 at the proximal end of the catheter 150 to adjust the rotation angle of the first gripper 220, and to pull the end of the third segment 123 away from the second segment 122 (or the fourth segment 141) at the proximal end of the catheter 150 to adjust the rotation angle of the second gripper 230. When it is required to detach the first control wire 120 from the mitral valve clip 200, the preset channel is adjusted to the non-closed state, and the operator pulls the end of first segment 121 that is away from the second segment 122 or pulls the end of the third segment 123 that is away from the second segment 122 (or the fourth segment 141) at the proximal end of the catheter 150, to achieve detachment of the first control wire 120 from the mitral valve clip 200.

Referring to Figs. 16 to 20, some embodiments of the present disclosure provide another prosthesis system including a delivery device and a prosthesis. The delivery device includes a second connecting shaft 410, a second central rod 420, and a first control wire. The prosthesis includes a second base 510 and a first movable member, where when the second central rod 420 is positioned at a coupling site between the second connecting shaft 410 and the second base 510, the second base 510 is coupled to the second connecting shaft 410. The first control wire is provided with a first segment for independently driving movement of the first movable member and a second segment, which are connected in sequence. When the second central rod 420 is positioned at the coupling site between the second connecting shaft 410 and the second base 510, the second central rod 420 forms a second preset channel 600 in a closed state at the coupling site with the second connecting shaft 410 and/or the second base 510, and the second segment is constrained within the second preset channel 600, thereby constraining the first segment at the coupling site. When the second central rod 420 is positioned outside the coupling site between the second connecting shaft 410 and the second base 510, the second preset channel 600 is in a non-closed state, and the first control wire can be detached from the second preset channel 600.

In this embodiment, the wall surface of the second preset channel 600 in the closed state is formed by two parts, one of which is provided by the second central rod 420, and the other of which is provided by the second connecting shaft 410 and/or the second base 510.

It should be noted that the prosthesis system provided in this embodiment is substantially the similar as the prosthesis system provided in the above embodiments, with the difference being that: the second preset channel 600 in the closed state in this embodiment is defined by the second central rod 420, and the second connecting shaft 410 and/or the second base 510. In addition, other contents described in the above embodiments can be applied to this embodiment in the case of no conflict. For example, the specific implementation of using the second central rod 420 to achieve the coupling between the second connecting shaft 410 and the second base 510 in this embodiment is similar to the specific implementation of using the first central rod 130 to achieve the coupling between the first connecting shaft 110 and the first base 210 described in the above embodiments, so the specific implementation of using the first central rod 130 to achieve the coupling between the first connecting shaft 110 and the first base 210 described in the above embodiments can be applied to this embodiment.

In some embodiments, a receiving channel is arranged on the second connecting shaft 410 and the second base 510 along the axial direction of the second connecting shaft 410. In this case, when the second central rod 420 is placed into the receiving channel and positioned at the coupling site between the second connecting shaft 410 and the second base 510, the second connecting shaft 410 remains stationary at least in the radial direction relative to the second base 510, thereby achieving the coupling between the second connecting shaft 410 and the second base 510. In some embodiments, a receiving channel is arranged on the second connecting shaft 410 and the second base 510 along the axial direction of the second connecting shaft 410, and the second connecting shaft 410 or the second base 510 is provided with a biased coupling portion. In this case, when the second central rod 420 is placed into the receiving channel and positioned at the coupling site between the second connecting shaft 410 and the second base 510, the second central rod 420 drives the biased coupling portion to be arranged along the axial direction of the second connecting shaft 410, thereby achieving the coupling between the second connecting shaft 410 and the second base 510.

Referring to Figs. 16 and 17, the second connecting shaft 410 includes a third contact surface 411. The second base 510 includes a fourth contact surface 511 for contacting at least partially the third contact surface 411 when the second connecting shaft 410 and the second base 510 are coupled. The third contact surface 411 is provided with a fourth through slot 412. The portion of the outer wall surface of the second central rod 420 corresponding to the fourth through slot 412 is a mating wall surface. When the second central rod 420 is positioned at the coupling site between the second connecting shaft 410 and the second base 510, at least part of the wall surface of the fourth through slot 412 and the mating wall surface of the second central rod 420 jointly form the second preset channel 600 in the closed state. When the second central rod 420 is positioned outside the coupling site between the second connecting shaft 410 and the second base 510, the wall surface of the fourth through slot 412 is separated from the mating wall surface of the second central rod 420. Thus, the second preset channel 600 to be in the non-closed state. Alternatively, the fourth through slot 412 is arranged on the fourth contact surface 511.

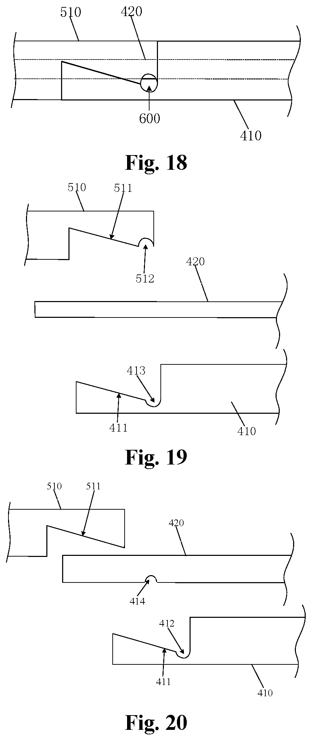

Referring to Figs. 18 and 19, the second connecting shaft 410 includes a third contact surface 411. The second base 510 includes a fourth contact surface 511 for contacting at least partially the third contact surface 411 when the second connecting shaft 410 and the second base 510 are coupled. The third contact surface 411 is provided with a fifth through slot 413, and the fourth contact surface 511 is provided with a sixth through slot 512. The portion of the outer wall surface of the second central rod 420 corresponding to the fifth through slot 413 and the sixth through slot 512 is a mating wall surface. When the second central rod 420 is positioned at the coupling site between the second connecting shaft 410 and the second base 510, the wall surface of the fifth through slot 413 and the wall surface of the sixth through slot 512 jointly form a fitting channel, which allows the first control wire to pass through. The mating wall surface is positioned within the fitting channel and forms the second preset channel 600 in the closed state together with part of the wall surface of the fitting channel. When the second central rod 420 is positioned outside the coupling site between the second connecting shaft 410 and the second base 510, the mating wall surface is positioned outside the fitting channel, thereby the second preset channel 600 to be in the non-closed state. Alternatively, the portion of the outer wall surface of the second central rod 420 corresponding to the fifth through slot 413 is a mating wall surface; or, the portion of the outer wall surface of the second central rod 420 corresponding to the sixth through slot 512 is a mating wall surface.

In this embodiment, there is an overlapping area between the fitting channel and the receiving channel. After the second central rod 420 is placed in the receiving channel, when the second central rod 420 is positioned at the coupling site between the second connecting shaft 410 and the second base 510, the second central rod 420 occupies at least part of the overlapping area. At this time, the mating wall surface is located in the fitting channel, and the part of the fitting channel not occupied by the second central rod 420 forms the second preset channel 600 in the closed state.

In some embodiments, referring to Fig. 20, the second connecting shaft 410 includes a third contact surface 411. The second base 510 includes a fourth contact surface 511 for contacting at least partially the third contact surface 411 when the second connecting shaft 410 and the second base 510 are coupled. The surface of the third contact surface 411 is provided with a fourth through slot 412. The portion of the outer wall surface of the second central rod 420 corresponding to the fourth through slot 412 is provided with a seventh through slot 414. When the second central rod 420 is positioned at the coupling site between the second connecting shaft 410 and the second base 510, at least part of the wall surface of the fourth through slot 412 and the wall surface of the seventh through slot 414 of the second central rod 420 jointly form the second preset channel 600 in the closed state. When the second central rod 420 is positioned outside the coupling site between the second connecting shaft 410 and the second base 510, the wall surface of the fourth through slot 412 is separated from the seventh through slot 414. Thus, the second preset channel 600 is in the non-closed state. Alternatively, the fourth through slot 412 may be arranged on the fourth contact surface 511, and the position of the seventh through slot 414 on the second central rod 420 may be adjusted accordingly.

It should be understood by those of ordinary skill in the art that the above embodiments are specific embodiments for implementing the present disclosure, and in practical applications, various changes may be made in form and detail thereto without departing from the spirit and scope of the present disclosure. Any person skilled in the art may make changes and modifications without departing from the spirit and scope of the present disclosure. The scope of protection of the present disclosure shall be subject to the scope defined by the claims.

## Claims

1. A prosthesis system, comprising a delivery device and a prosthesis;
the delivery device comprising a first connecting shaft and a first control wire;
the prosthesis comprising a first base and a first movable member, wherein the first base is detachably coupled to the first connecting shaft, forming a first preset channel at a coupling site;
the first control wire being provided with a first segment for independently driving movement of the first movable member and a second segment, the first segment and the second segment being connected in sequence;
wherein when the first connecting shaft and the first base are coupled, the first preset channel is in a closed state, and the second segment is constrained within the first preset channel, thereby constraining the first segment at the coupling site; and when the first connecting shaft and the first base are detached, the first preset channel is in a non-closed state, and the first control wire is detachable from the first preset channel.

2. The prosthesis system according to claim 1, wherein
the first connecting shaft comprises a first contact surface;
the first base comprises a second contact surface for at least partially contacting the first contact surface when the first connecting shaft and the first base are coupled; and
the first preset channel is positioned between the first contact surface and the second contact surface.

3. The prosthesis system according to claim 2, wherein
a surface of the first contact surface or a surface of the second contact surface is provided with a first through slot;
wherein when the first base is coupled to the first connecting shaft, a wall surface of the first through slot and a corresponding portion of the other contact surface jointly form the first preset channel in the closed state; and
when the first base is detached from the first connecting shaft, the wall surface of the first through slot is detached from the other contact surface, causing the first preset channel to be in the non-closed state.

4. The prosthesis system according to claim 2, wherein
a surface of the first contact surface is provided with a second through slot, and a surface of the second contact surface is provided with a third through slot;
wherein when the first base is coupled to the first connecting shaft, a wall surface of the second through slot and a wall surface of the third through slot jointly form the first preset channel in the closed state; and
when the first base is detached from the first connecting shaft, the wall surface of the second through slot is detached from the wall surface of the third through slot, causing the first preset channel to be in the non-closed state.

5. The prosthesis system according to claim 3 or 4, wherein
the first contact surface and the second contact surface are such configured that a contact portions between the second contact surface and the first contact surface forms a constraint such that the first connecting shaft is driven to move relative to the first base along a radial direction of the first connecting shaft when the first connecting shaft moves away from the first base along an axial direction of the first connecting shaft.

6. The prosthesis system according to claim 5, wherein
the first contact surface comprises a first distal blocking surface at a distal end, a first proximal blocking surface at a proximal end, and a first inclined surface arranged between the first distal blocking surface and the first proximal blocking surface; the second contact surface comprises a second distal blocking surface at a distal end for corresponding to the first distal blocking surface, a second proximal blocking surface at a proximal end for corresponding to the first proximal blocking surface, and a second inclined surface arranged between the second distal blocking surface and the second proximal blocking surface and for being attached to the first inclined surface; and
a maximum vertical distance from the first inclined surface to an edge of the first connecting shaft decreases as a distance to the distal end of the first base increases.

7. The prosthesis system according to claim 6, wherein
the first inclined surface is an inclined surface, a curved surface, or a wavy surface.

8. The prosthesis system according to claim 3 or 4, wherein
the first connecting shaft is provided with a first coupling portion, the first base is provided with a second coupling portion, and the first coupling portion or the second coupling portion is biased from an axial direction of the first connecting shaft; and
when the biased coupling portions are driven to be arranged along the axial direction of the first connecting shaft, the first coupling portion and the second coupling portion are coupled, with the first contact surface arranged on a side of the first coupling portion facing the second coupling portion, and the second contact surface arranged on a side of the second coupling portion facing the first coupling portion.

9. The prosthesis system according to claim 8, wherein
one of the first coupling portion and the second coupling portion is provided with a first protrusion, and the other is provided with a first groove;
wherein when the first coupling portion or the second coupling portion is biased from the axial direction of the first connecting shaft, the first protrusion is disengaged from the first groove; and when the biased coupling portions are driven to be arranged along the axial direction of the first connecting shaft, the first protrusion is received in the first groove.

10. The prosthesis system according to claim 9, wherein
the first coupling portion further comprises an inwardly biased support, the first protrusion is arranged on a distal end of the support and protrudes outwardly perpendicular to an axial direction of the support; the first base is provided with a second receiving channel for receiving the first coupling portion, and the first groove is provided on a wall surface of the second receiving channel; when the support is driven to deflect so that an axis of the support is parallel to an axis of the connecting shaft, the first protrusion is received in the first groove;
or,
the first coupling portion further comprises an outwardly biased support, the first protrusion is arranged on a distal end of the support and protrudes inwardly perpendicular to an axial direction of the support; the first groove is arranged on an outer wall surface of the first base; when the support is driven to deflect so that an axis of the support is parallel to an axis of the connecting shaft, the first protrusion is received in the first groove.

11. The prosthesis system according to claim 3 or 4, wherein
one of the first contact surface and the second contact surface is provided with a second protrusion, and the other is provided with a second groove, wherein a protruding direction of the second protrusion and a recessing direction of the second groove are both in a radial direction of the connecting shaft; and
when the first contact surface is at least partially in contact with the second contact surface, the second protrusion is fitted into the second groove.

12. The prosthesis system according to claim 5, wherein
the delivery device further comprises: a first central rod, wherein the first central rod is configured such that when the first central rod is positioned at the coupling site between the first connecting shaft and the first base, and the first connecting shaft maintains stationary at least in a radial direction relative to the first base, thereby achieving coupling between the first connecting shaft and the first base.

13. The prosthesis system according to claim 8, wherein
the delivery device further comprises: a first central rod, wherein the first central rod is configured such that when the first central rod is positioned at the coupling site between the first connecting shaft and the first base, and the first central rod drives the biased coupling portion to be arranged along the axial direction of the first connecting shaft.

14. The prosthesis system according to any one of claims 1 to 13, wherein
a diameter of the second segment is larger than a diameter of the first segment, or
the first control wire further comprises a third segment connected to an end of the second segment that is away from the first segment, and a diameter of the second segment is larger than a diameter of each of the first segment and the third segment, or
the first control wire further comprises a third segment connected to an end of the second segment that is away from the first segment, and a diameter of the second segment is smaller than a diameter of a portion of each of the first segment and the third segment that is connected the second segment.

15. The prosthesis system according to any one of claims 1 to 13, wherein
the prosthesis further comprises a second movable member;
the first control wire further comprises a third segment connected to an end of the second segment that is away from the first segment, and the third segment is configured to independently drive the second movable member to move;
wherein when the first connecting shaft and the first base are coupled, the first preset channel is in the closed state, and the second segment is constrained within the first preset channel, thereby constraining the third segment at the coupling site.

16. The prosthesis system according to claim 15, wherein
a radial dimension of a portion of each of the first segment and the third segment that is connected to the second segment is larger than a radial dimension of the second segment, a minimum radial dimension of the first preset channel in the closed state is larger than or equal to the radial dimension of the second segment, and a maximum radial dimension of the first preset channel in the closed state is smaller than the radial dimension of the portion of each of the first segment and the third segment that is connected to the second segment;
or,
a radial dimension of each of the first segment and the third segment is smaller than a radial dimension of the second segment, a maximum radial dimension of the first preset channel in the closed state is larger than or equal to the radial dimension of the second segment, a minimum radial dimension of the first preset channel in the closed state is smaller than or equal to the radial dimension of the second segment, and the maximum radial dimension of the first preset channel in the closed state is not equal to the minimum radial dimension thereof.

17. The prosthesis system according to any one of claims 1 to 13, wherein
the prosthesis further comprises a second movable member;
the delivery device further comprises a second control wire; and the second control wire is provided with a third segment for independently driving movement of the second movable member and a fourth segment, wherein the third segment and the fourth segment are connected in sequence;
wherein when the first preset channel is in the closed state, the fourth segment is constrained within the first preset channel, thereby constraining the third segment and the fourth segment at the coupling site; and when the first preset channel is in the non-closed state, the second control wire is detachable from the first preset channel.

18. The prosthesis system according to claim 17, wherein
when the first preset channel is in the closed state, a portion of the first preset channel positioned on a side of the second segment close to the first segment is a first portion, and a portion on the other side is a second portion, the second portion is positioned on a side of the fourth segment close to the third segment;
the radial dimension of the second segment is larger than the radial dimension of the first segment and larger than a radial dimension of the first portion of the first preset channel in the closed state; and
a radial dimension of the fourth segment is larger than a radial dimension of the third segment and larger than a radial dimension of the second portion of the first preset channel in the closed state.

19. The prosthesis system according to any one of claims 15 to 18, wherein
the prosthesis is a mitral valve clip or tricuspid valve clamp;
the first movable member is a first gripper, and the second movable member is a second gripper, the first and second grippers rotate about an axis of the first base, and
the first segment is movably connected to the first gripper, and the second segment is movably connected to the second gripper.

20. The prosthesis system according to claim 19, wherein
the first segment extends distally from the first preset channel, and after extending to a position of the first gripper, extends back towards a proximal end of the delivery device, forming a curved portion;
the curved portion is configured to interpenetrate with the first gripper or with an attachment arranged on the first gripper.

21. The prosthesis system according to claim 20, wherein
the attachment is a fixing wire, wherein the fixing wire is arranged on the first gripper and configured to form a wire-body for interpenetrating with the curved portion.

22. The prosthesis system according to claim 21, wherein
the first gripper is provided with a first through hole, the fixing wire is provided with a fixing knot, the wire-body is connected to the fixing knot and is provided with a second through hole by winding around the fixing knot, the wire-body passes through the first through hole from a side of the first gripper that is away from the first base, the fixing knot is fixed on the side of the first gripper that is away from the first base, and the fixing knot is configured to be unable to pass through the first through hole.

23. A prosthesis system, comprising a delivery device and a prosthesis;
wherein the delivery device comprises a second connecting shaft, a second central rod, and a first control wire;
the prosthesis comprises a second base and a first movable member, wherein when the second central rod is positioned at a coupling site between the second connecting shaft and the second base, the second base is coupled to the second connecting shaft;
the first control wire is provided with a first segment for independently driving movement of the first movable member and a second segment, the first segment and the second segment are connected in sequence; and
wherein when the second central rod is positioned at the coupling site between the second connecting shaft and the second base, the second central rod forms a second preset channel in a closed state at the coupling site with the second connecting shaft and/or the second base, and the second segment is constrained within the second preset channel, thereby constraining the first segment at the coupling site; and when the second central rod is positioned outside the coupling site between the second connecting shaft and the second base, the second preset channel is in a non-closed state, and the first control wire is detachable from the second preset channel.

24. The prosthesis system according to claim 23, wherein
the second connecting shaft comprises a third contact surface;
the second base comprises a fourth contact surface for at least partially contacting the third contact surface when the second connecting shaft and the second base are coupled;
the third contact surface or the fourth contact surface is provided with a fourth through slot; and
a portion of an outer wall surface of the second central rod corresponding to the fourth through slot is a mating wall surface;
wherein when the second central rod is positioned at the coupling site between the second connecting shaft and the second base, at least part of a wall surface of the fourth through slot and the mating wall surface of the second central rod jointly form the second preset channel in the closed state; and
when the second central rod is positioned outside the coupling site between the second connecting shaft and the second base, the wall surface of the fourth through slot is separated from the mating wall surface of the second central rod, causing the second preset channel to be in the non-closed state.

25. The prosthesis system according to claim 23, wherein
the second connecting shaft comprises a third contact surface;
the second base comprises a fourth contact surface for at least partially contacting the first contact surface when the second connecting shaft and the second base are coupled;
the third contact surface is provided with a fifth through slot, and the fourth contact surface is provided with a sixth through slot; and
a portion of the outer wall surface of the second central rod corresponding to the fifth through slot and/or the sixth through slot is a mating wall surface;
wherein when the second central rod is positioned at the coupling site between the second connecting shaft and the second base, a wall surface of the fifth through slot and a wall surface of the sixth through slot jointly form a fitting channel, which allows the first control wire to pass through, wherein the mating wall surface is positioned within the fitting channel and forms the second preset channel in the closed state together with part of the wall surface of the fitting channel; and
when the second central rod is positioned outside the coupling site between the second connecting shaft and the second base, the mating wall surface is positioned outside the fitting channel, causing the second preset channel to be in the non-closed state.

26. The prosthesis system according to claim 23, wherein
the second connecting shaft comprises a third contact surface;
the second base comprises a fourth contact surface for at least partially contacting the third contact surface when the second connecting shaft and the second base are coupled;
a surface of the third contact surface or a surface of the fourth contact surface is provided with a fourth through slot; and
a portion of the outer wall surface of the second central rod corresponding to the fourth through slot is provided with a seventh through slot;
wherein when the second central rod is positioned at the coupling site between the second connecting shaft and the second base, a wall surface of the fourth through slot and a wall surface of the seventh through slot jointly form the second preset channel in the closed state; and
when the second central rod is positioned outside the coupling site between the second connecting shaft and the second base, the wall surface of the fourth through slot is separated from the wall surface of the seventh through slot, causing the second preset channel to be in the non-closed state.
